# NEW EUROPEAN PATENT SPECIFICATION

(11) **EP 1 589 973 B2**
(45) Date of publication and mention of the opposition decision: **26.12.2012**
(45) Mention of the grant of the patent: 07.04.2010
(21) Application number: 04704524.0
(22) Date of filing: 23.01.2004
(51) Int. Cl.: A61K 31/517

(54) **FORMULATION AND METHODS FOR THE TREATMENT OF THROMBOCYTHEMIA**
FORMULIERUNG UND VERFAHREN ZUR BEHANDLUNG VON THROMBOCYTHÄMIE
FORMULATION ET METHODES DE TRAITEMENT DE LA THROMBOCYTHEMIE

(30) Priority: 23.01.2003 US 441765 P
(43) Date of publication of application: 02.11.2005
(73) Proprietor: Shire Biopharmaceuticals Holdings Ireland Limited, St Helier JE4 8PX (GB)
(72) Inventor: FRANKLIN, Richard, Fleet, Hampshire GU51 4NQ (GB)
(74) Representative: Atkinson, Jonathan David Mark
(86) International application number: PCT/CA2004/000096
(87) International publication number: WO 2004/064841

(56) References cited:
- WO-A-93/09794
- WO-A1-93/08798
- US-A- 5 306 709
- US-A- 6 156 753
- US-A1- 2002 004 065
- US-B1- 6 221 383
- STOREN, E. ET AL.: "Long-term use of anagrelide in young patients with essential thrombocythemia" BLOOD, vol. 97, no. 4, 15 February 2001 (2001-02-15), pages 863-866, XP002281471
- HARTMUND DERENDORF ET AL.: 'Pharmakokinetik', vol. 2, 2002, WISSENSCHAFTLICHE VERLAGGESELLSCHAFT MBH, STUTTGART, DE, ISBN 3804719074 article 'Einführung in die Theorie und Relevanz für die Artneimitteltherapie', pages 121 - 124
- AARON TOMER: 'Effects of anagrelide on in vivo megakaryocyte proliferation and maturation in essential thrombocythemia' BLOOD vol. 99, no. 5, 01 March 2002, pages 1602 - 1609

## Description

### FIELD OF THE INVENTION

The present invention relates to methods for treating thrombocythemia. The present invention also relates to formulations that are useful for reducing platelet counts.

### BACKGROUND OF THE INVENTION

Thrombocythemia is a chronic disorder associated with increased or abnormal production of blood platelets. Since platelets are involved in blood clotting their abnormal production can result in the inappropriate formation of blood clots or in bleeding, with the consequence that patients' risk of gastrointestinal bleeding, heart attack and stroke is increased.

Anagrelide, a quinazoline derivative phosphodiesterase inhibitor, was first described as a blood platelet anti-aggregative agent, anti-hypertensive agent and bronchodilatator agent in US patent 3,932,407 issued January 13, 1976 and in Reissue patent No. Re.31,617 issued June 26, 1984.

Anagrelide is used currently for the treatment of essential thrombocythemia and various other myeloproliferative disorders. Anagrelide was approved and launched in 1997 for the treatment of essential thrombocythemia in the US and Canada. In December 1998, the US FDA approved an expanded label for anagrelide; specifically, for the treatment of patients with thrombocythemia secondary to myeloproliferative disorders, including polycythemia vera (PV) and chronic myelogenous leukemia (CML).

WO 93/097 94, US 5 306 709 and E.C. Storen et al., Blood, vol. 97, no. 4, pages 863-866, 2001 disclose the use of anagrelide for oral or parenteral treatment of thrombocythemia.

Anagrelide is available as 0.5mg and 1.0 mg capsule for oral administration. The most common adverse event observed with anagrelide are related to vasodilatory and positive inotropic effect. These include, headache, diarrhea, palpitations, and tachycardia.

It would therefore be desirable to have other formulations that could be used for treating thrombocythemia.

### SUMMARY OF THE INVENTION

As set forth in US patent 3,932,407 issued January 13, 1976 and in Reissue patent No. Re.31,617 June 26, 1984 quinazoline derivative including Aanagrelide can be prepared in a solid form for oral and/or parenteral use as blood platelet anti-aggregative agents and/or anti-hypertensive agents and/or bronchodilatator agents. However, the patent does not suggest that it would be desirable to avoid the first pass metabolism through the liver in order to reduce some of anagrelide side-effects when administered orally. The patent also does not suggest that it would be possible or desirable to prepare a transdermal formulation or to use the formulation for the treatment or prevention of thrombocythemia.

Without being bound to any theory (an understanding of the mechanism is not necessary to practice the present invention, and the present invention is not limited to any particular mechanism), Applicants believe that certain cardiovascular or inotropic related side effects are associated with a metabolite as a result of first pass through the liver. In accordance with this invention the inventors have found that surprisingly, certain of these side effects can be reduced by avoiding the first pass liver metabolism.

Applicants have discovered that the transdermal formulation of this invention provides surprising beneficial effects.

Applicant have determined that anagrelide can be effectively administered transdermally.

In one embodiment, the formulations of this invention provide consistent dosage of the active ingredient

In one embodiment, the formulations of this invention achieve sustained plasma concentration of the pharmaceutically active agent.

In one embodiment, the formulations of this invention encourage patient compliance.

In one aspect, the present invention provides the use of a formulation comprising as an active ingredient an effective amount of anagrelide in the preparation of a medicament for use in the treatment of thrombocythemia in a host wherein the mode of administration is by transdermal administration. Also disclosed is the preparation of a medicament as described above for reducing the side effects associated with anagrelide.

### DESCRIPTION OF THE FIGURES

Figure 1 represents the mean plasma concentration-time profiles of anagrelide and Metabolite A after 1mg orally and after dermal application of a saturated solution for 24h.
Figure 2 represents the effectiveness of continuous low-level exposure to anagrelide.

### DETAILED DESCRIPTION OF THE INVENTION

In one embodiment, formulation of the present invention comprise those wherein the following embodiments are present, either independently or in combination.

Anagrelide has been administered to human subjects as a capsule formulation. Such tablet formulation of anagrelide can be associated with undesired effects when administered to a group of subjects. Surprisingly, the presently claimed transdermal formulations minimize or eliminate such effects while maintaining a consistent, desirable plasma concentration of the pharmacologically active agent.

In one embodiment anagrelide, anagrelide in base form, or a pharmaceutically acceptable salt of anagrelide is administered transdermally.

In one embodiment, anagrelide, anagrelide in base form, or a pharmaceutically acceptable salt of anagrelide is in the form of reservoir formulation.

In one embodiment, anagrelide, anagrelide in base form, or a pharmaceutically acceptable salt of anagrelide is in the form of a single layer formulation comprising anagrelide, anagrelide in base form, or a pharmaceutically acceptable salt of anagrelide and at least one adhesive.

In one embodiment, anagrelide, anagrelide in base form, or a pharmaceutically acceptable salt of anagrelide is in the form of a multiple layer formulation wherein at least one layer of said multiple layer formulation comprises anagrelide, anagrelide in base form, or a pharmaceutically acceptable salt of anagrelide and at least one adhesive.

In one embodiment, anagrelide, anagrelide in base form, or a pharmaceutically acceptable salt of anagrelide is in the form of a matrix formulation.

In one embodiment, anagrelide, anagrelide in base form, or a pharmaceutically acceptable salt of anagrelide is administered in an amount of 0.01 to 20 mg/kg/day.

In one embodiment, anagrelide, anagrelide in base form, or a pharmaceutically acceptable salt of anagrelide is administered in a daily dose 0.5 to 10 mg

In one embodiment, anagrelide, anagrelide in base form, or a pharmaceutically acceptable salt of anagrelide is administered in a daily dose 0.5 to 3 mg.

In one embodiment, anagrelide, anagrelide in base form, or a pharmaceutically acceptable salt of anagrelide is administered in a daily dose 1 to 2 mg.

In one embodiment, anagrelide, anagrelide in base form, or a pharmaceutically acceptable salt of anagrelide is in the form of a composition which further comprises at least one skin permeation enhancer.

In one embodiment, there is provided, a use in accordance with this invention wherein administration is via a transdermal patch having a single-layer drug-in-adhesive system comprising a composition containing anagrelide, anagrelide in base form, or a pharmaceutically acceptable salt of anagrelide, any optional excipients, and at least one skin-contacting adhesive, which is combined with a single backing film

In one embodiment, there is provided, a use in accordance with this invention, wherein administration is via a transdermal patch having a multi-layer drug-in-adhesive system wherein: (a) said system comprises at least two distinct layers comprising at anagrelide, anagrelide in base form, or a pharmaceutically acceptable salt of anagrelide and at least one adhesive, and a membrane between said at least two layers or (b) said system comprises at least two distinct layers comprising at anagrelide, anagrelide in base form, or a pharmaceutically acceptable salt of anagrelide and at least one adhesive, and a single backing film.

In one embodiment, there is provided, a use in accordance with this invention, wherein administration is via a transdermal patch having a reservoir transdermal system comprising a liquid compartment containing a solution or suspension of anagrelide, anagrelide in base form, or a pharmaceutically acceptable salt of anagrelide, a release liner, and between said release liner and said liquid compartment, a semi-permeable membrane and at least one adhesive.

In one embodiment, there is provided, a use in accordance with this invention, wherein administration is via a transdermal patch having a matrix system comprising a semisolid matrix containing a solution or suspension of anagrelide, anagrelide in base form, or a pharmaceutically acceptable salt of anagrelide which is in direct contact with a release liner, and a skin adhesion component incorporated in an overlay which forms a concentric configuration around said semisolid matrix.

In one embodiment, there is provided, a use in accordance with this invention, wherein administration is via a transdermal patch containing anagrelide, anagrelide in base form, or a pharmaceutically acceptable salt of anagrelide intimately distributed in a matrix.

In one embodiment, there is provided, a use in accordance with this invention, wherein administration is via a transdermal patch containing 1 mg to 100 mg of anagrelide, anagrelide in base form, or a pharmaceutically acceptable salt of anagrelide per patch.

In one embodiment, there is provided, a use in accordance with this invention, wherein administration is via a transdermal patch containing an amount of anagrelide, anagrelide in base form, or a pharmaceutically acceptable salt of anagrelide sufficient to provide a daily dose of 0.5 to 3 mg.

In one embodiment, there is provided, a use in accordance with this invention, wherein administration is via a transdermal patch containing a composition comprising anagrelide, anagrelide in base form, or a pharmaceutically acceptable salt of anagrelide and an acrylic adhesive.

In one embodiment, there is provided, a use in accordance with this invention, wherein administration is via a transdermal patch containing having an area of 5cm² to 100cm².

In one embodiment, there is provided a use in accordance with this invention, wherein anagrelide, anagrelide in base form, or a pharmaceutically acceptable salt of anagrelide is administered over a period of time of 1 to 7 days.

In one embodiment, there is provided, a use in accordance with this invention, wherein anagrelide, anagrelide in base form, or a pharmaceutically acceptable salt of anagrelide is administered over a period of time of 3 to 4 days.

In one embodiment, there is provided, a use in accordance with this invention, wherein anagrelide in base form is administered.

There is also disclosed a method in accordance with this invention, comprises:
(b) contacting said area of skin with a source of skin permeable form of anagrelide, anagrelide in base form, or a pharmaceutically acceptable salt of anagrelide; and
(b) maintaining said source in material transmitting relationship to said area of skin for a period of at least 24 hours.

There is also disclosed the reduction of side effects associated with the oral administration of anagrelide comprises administering to a patient in need thereof anagrelide, anagrelide in base form, or a pharmaceutically acceptable salt of anagrelide in a manner whereby first pass liver metabolism is avoided.

In one aspect, there is provided, a non-oral, pharmaceutical composition comprising anagrelide, anagrelide in base form, or a pharmaceutically acceptable salt of anagrelide and at least one skin permeation enhancer.

In one aspect, there is provided, a non-oral, pharmaceutical composition comprising anagrelide, anagrelide in base form, or a pharmaceutically acceptable salt of anagrelide and at least one adhesive.

In one aspect, there is provided, a composition according to the invention wherein at least one adhesive is an acrylic adhesive.

This disclosure provides a use for treating thrombocythemia with minimal undesired effects comprising administering anagrelide transdermally or subdermally (implant).

In one embodiment, the thrombocythemia is associated with myeoloproliferative blood disorders.

In one embodiment, the thrombocythemia is associated with essential thrombocythemia (ET), chronic myologenous leukemia (CML), polycythemia vera (PV), agnogenic myeloid metaplasia (AMM) or sickle cell anemia(SCA).

In a further embodiment;
The thrombocythemia is caused by ET.
The thrombocythemia is caused by CML.
The thrombocythemia is caused by PV.
The thrombocythemia is caused by AMM.
The thrombocythemia is caused by SCA.

The formulations can be used to reduce platelet count in a host.

By the term pharmaceutically acceptable salts or ion pairs of anagrelide are meant those derived from pharmaceutically acceptable inorganic and organic acids and bases. Examples of suitable acids include hydrochloric, hydrobromic, sulphuric, nitric, perchloric, fumaric, maleic, phosphoric, glycollic, lactic, salicylic, succinic, toluene-p-sulphonic, tartaric, acetic, citric, methanesulphonic, formic, benzoic, malonic, naphthalene-2-sulphonic and benzenesulphonic acids. Other acids such as oxalic, while not in themselves pharmaceutically acceptable, may be useful as intermediates in obtaining the compounds of the invention and their pharmaceutically acceptable acid addition salts.

Compounds disclosed in the present application can be prepared by methods well know in the art, see for example US patents 3,932,407, 5,801,245 and 6,388,073. The compounds can also be obtained from chemical supply companies such as Sigma.

Unless otherwise defined, all technical and scientific terms used herein have the same meaning as commonly understood by one of ordinary skill in the art to which this invention belongs. In case of conflict, the present specification including definitions, will control. In addition, the materials, methods, and examples are illustrative only and not intended to be limiting.

It will be appreciated that the amount of a compound of the disclosure required for use in treatment will vary not only the nature of the condition for which treatment is required and the age and condition of the patient and will be ultimately at the discretion of the attendant physician or veterinarian. In general however a suitable dose will be in the range of from about 0.01 to about 20 mg/kg of body weight per day, preferably in the range of 0.05 to 10 mg/kg/day, most preferably in the range of .04 to 5 mg/kg/day. In a further embodiment, the daily dose will be between 0.5 and 15 mg daily. In a further embodiment, the daily dose will be between 0.5 and 12 mg daily. In a further embodiment, the daily dose will be between 0.5 and 10 mg daily. In a further embodiment, the daily dose will be between 0.5 and 5 mg daily. In a further embodiment, the daily dose will be between 1 and 4 mg daily In a further embodiment, the daily dose will be between 0.5 and 3 mg daily. In a further embodiment, the daily dose will be between 1 and 3 mg daily In a further embodiment, the daily dose will be between 1 and 2 mg daily.

In accordance with one aspect of this invention the first pass through the liver can be avoided by administering anagrelide by using, transdermal administration.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or colouring agents. Transdermal patches include but are not limited to:
1. **Single-layer Drug-in-Adhesive system** is characterized by the inclusion of the drug directly within the skin-contacting adhesive. In this transdermal system design, the adhesive not only serves to affix the system to the skin, but also serves as the formulation foundation, containing the drug and all the excipients under a single backing film.
2. **The Multi-layer Drug-in-Adhesive** is similar to the Single-layer Drug-in-Adhesive in that the drug is incorporated directly into the adhesive. However, the multi-layer encompasses either the addition of a membrane between two distinct drug-in-adhesive layers or the addition of multiple drug-in-adhesive layers under a single backing film.
3. **The Reservoir transdermal system design** is characterized by the inclusion of a liquid compartment containing a drug solution or suspension separated from the release liner by a semi-permeable membrane and adhesive. The adhesive component of the product responsible for skin adhesion can either be incorporated as a continuous layer between the membrane and the release liner or in a concentric configuration around the membrane
4. **The Matrix system design** is characterized by the inclusion of a semisolid matrix containing a drug solution or suspension which is in direct contact with the release liner. The component responsible for skin adhesion is incorporated in an overlay and forms a concentric configuration around the semisolid matrix.

The patches of this disclosure are matrix or monolithic-type laminated structures. Such transdermal patches are well known in the art They comprise a matrix layer of the drug(s) admixed with a pressure sensitive adhesive and a backing layer. The matrix serves as both the drug reservoir and the means by which the patch is affixed to the skin. Prior to use, the patch will also include an impermeable release liner layer.

The backing layer is impermeable to the drug and other components of the matrix and defines the top face surface of the patch. It may be made of a single layer or film of polymer, or be a laminate of one or more polymer layers and metal foil. Examples of polymers suitable for use in making backing films are polyvinylchloride, polyvinylidene chloride, polyolefins such as ethylene-vinyl acetate copolymers, polyethylene, and polypropylene, polyurethane, and polyesters such as polyethylene terephthalate.
The pressure-sensitive adhesive of the matrix will normally be a solution polyacrylate, a silicone, or polyisobutylene (PIB). Such adhesives are well known in the transdermal art. See, for instance, the Handbook of Pressure Sensitive Adhesive Technology, 2nd Edition (1989) Van Nostrand, Reinhold.

Pressure sensitive solution polyacrylate adhesives are made by copolymerizing one or more acrylate monomers ("acrylate" is intended to include both acrylates and methacrylates), one or more modifying monomers, and one or more functional group-containing monomers in an organic solvent. The acrylate monomers used to make these polymers are normally alkyl acrylates of 4-17 carbon atoms, with 2-ethylhexyl acrylate, butyl acrylate, and isooctyl acrylate being preferred. Modifying monomers are typically included to alter the Tg of the polymer. Such monomers as vinyl acetate, ethyl acrylate and methacrylate, and methyl methacrylate are useful for this purpose. The functional group-containing monomer provides sites for crosslinking. The functional groups of these monomers are preferably carboxyl, hydroxy or combinations thereof. Examples of monomers that provide such groups are acrylic acid, methacrylic acid and hydroxy-containing monomers such as hydroxyethyl acrylate. The polyacrylate adhesives are preferably crosslinked using a crosslinking agent to improve their physical properties, (e.g., creep and shear resistance). The crosslinking density should be low since high degrees of crosslinling may affect the adhesive properties of the copolymer adversely. Examples of crosslinking agents are disclosed in U.S. Pat. No. 5,393,529. Solution polyacrylate pressure sensitive adhesives are commercially available under tradenames such as GELVA.TM. and DURO-TAK.TM. from 3M.

Polyisobutylene adhesives are mixtures of high molecular weight (HMW) PIB and low molecular weight (LMW) PIB. Such mixtures are described in the art, e.g., PCT/US91/02516. The molecular weight of the HMW PIB will usually be in the range of about 700,000 to 2,000,000 Da, whereas that of the LMW PIB will typically range between 35,000 to 60,000. The molecular weights referred to herein are weight average molecular weight. The weight ratio of HMW PIB to LMW PEB in the adhesive will normally range between 1:1 to 1:10. The PIB adhesive will also normally include a tackifier such as polybutene oil and high Tg, low molecular weight aliphatic resins such as the ESCOREZ.TM. resins available from Exxon Chemical. Polyisobutylene polymers are available commercially under the tradename VISTANEX.TM. from Exxon Chemical.

The silicone adhesives that may be used in forming the matrix are typically high molecular weight polydimethyl siloxanes or polydimethyldiphenyl siloxanes. Formulations of silicone adhesives that are useful in transdermal patches are described in U.S. Pat Nos. 5,232,702, 4,906,169 and 4,951,522.

In addition to the pressure sensitive adhesive and anagrelide, the matrix will typically contain sufficient amounts of permeation enhancers to increase the permeability of the anagrelide through the skin. Examples of skin permeation enhancers that may be included in the matrix are described above. The amount of permeation enhancer included in the matrix will depend upon the particular enhancer(s) used. In most instances then enhancer will constitute in the range of 1 to 20% by weight of the matrix.

The matrix may contain other additives depending upon the particular adhesive used. For instance, materials, such as polyvinyl pyrrolidone (PVP), that inhibit drug crystallization, hygroscopic agents that improve the duration of wear, or additives that improve the physical (e.g., cold flow) or adhesive (e.g., tack, cohesive strength) properties of the matrix may be included.

The patches of the disclosure may be fabricated using procedures known in the transdermal patch art. The procedure will generally involve formulating the matrix (i.e., mixing the adhesive, drug(s), permeation enhancer, and additives, if any), casting the matrix onto the backing or release liner layer, removing solvent from the matrix and applying the backing/release liner layer as the case may be. As is apparent to those of skill in the art, the matrix composition having an effective amount of the drug dispersed therein can be incorporated into various transdermal constructions and therefore, applicants are not limited to the embodiments exemplified below.

In a further embodiment, anagrelide can be administered trandermally using a metered dose transdermal spray. In such system, the patient simply positions a unit comprising the active agent against the skin and activates the proper command to spray a small accurate volume of liquid comprising the active agent onto a defined area of skin. The Liquid evaporates leaving an invisible water resistant deposit from which the drug is absorbed into the body. For example technology known as the Acrux^{tm} technology can be used.

Percutaneous or transdermal delivery of pharmacologically active agents has become feasible in recent years largely due to vehicles therefore which allow increased permeation of said agents into the body surface to which applied. Such agents which may be useful for the preparation of transdermal formulation of this invention include, but are not necessarily limited to, dimethylsulfoxide (U.S. Pat. No. 3,551,554); various 1-substituted azacycloalkan-2-ones such as azone (U.S. Pat. Nos. 4,562,075, 4,405,616, 4,326,893 and 3,989,816); sugar esters in combination with sulfoxide or phosphine oxide (U.S. Pat. Nos. 4,130,667, 4,130,643, 4,046,836, 3,952,099, and 3,896,238); lower alkyl amides (U.S. Pat. No. 3,472,931); certain aliphatic sulfoxides (U.S. Pat. No. 3,903,256); a. composition containing glycerol monooleate, ethanol and isopropyl myristate (U.S. Pat. No. 4,335,115); a binary mixture of 1-dodecylazacycloheptan-2-one and a compound selected from a diol or a second N-substituted azacycloalkyl-2-one (U.S. Pat. No. 4,557,934); and polyethylene glycol monolaurate (U.S. Pat. No. 4,568,343). U.S. Pat Nos. 3,551,554, 4,562,075, 4,405,616, 4,326,893; 3,989,816, 4,130,667, 4,130,643, 4,046,886, 3,952,099, 3,896,238, 3,472,931, 3,903,256, 4,335,115, 4,557,934, and 4,568,343.

It is contemplated that the transdermal or implant formulations of this disclosure will find utility in both humans and animals, i.e., will have both medical and veterinary applications for providing increased percutaneous absorption of the pharmaceutically active agent. As used herein, the term "percutaneous" refers to the passage of such agents through skin (typically intact).

The transdermal formulations of the present disclosure may be administered using a variety of devices which have been described in the art. For example, such devices include, but are not limited to those described in U.S. Pat. Nos. 3,598,122, 3,598,123, 3,710,795, 3,731,683, 3,742,951, 3,814,097, 3,921,636, 3,972,995, 3,993,072, 3,993,073, 3,996,934, 4,031,894, 4,060,084, 4,069,307, 4,077,407, 4,201,211,4,230,105,4,292,299, and 4,292,303. The dosage forms of the present invention may incorporate certain pharmaceutically acceptable excipients, which are conventional in the art. These include, but are not limited to, gelling agents, cream and ointment bases, and the like

The compound shall be present in the claimed dosage forms in an effective amount. The term "an effective amount" shall refer to an amount calculated to achieve and maintain blood levels which will bring about the desired beneficial or therapeutic effect over the period of time desired. These amounts will vary depending upon the amount of pharmacologically active agent required to achieve the desired beneficial or therapeutic effect, whether one or more patches will be administered simultaneously the specific formulation of the patch, the age and condition of the patient to be treated, and the like. Such conventional dosage titration techniques, familiar to the skilled artisan, may be utilized to determine the amount of a anagrelide present in the ultimate pharmaceutical dosage form for any specific situation. Typically, an effective amount is between about 1 mg to about 100 mg of compound per patch. More preferably, the effective amount is between about 1 mg to about 50 mg of compound. In a further embodiment, the amount of anagrelide per patch will be adjusted to provide a daily dose of about 0.5 to 2 mg daily and preferably from about 1 to 2 mg daily. The effective amount may be between about 1 mg and about 300 mg of compound for the transdermal patch formulation. The amount actually contained in the patch will depend on the factors described as well as the days of treatment provided per patch.

The pharmacologically active compound is administered by known technique such as placing the patch containing said agent and transdermal formulation therefore on a body surface and maintaining said source on said body surface in agent and composition transmitting relation thereto.

One of the transdermal formulations of this invention utilizes ethanol, water, azone, and optionally propylene glycol to enhance the permeation of the pharmacologically anagrelide. As noted supra, azone is known to be useful for transdermal permeation enhancement and is chemically 1-dodecylazacyloheptan-2-one. Azone can be prepared as described in U.S. Pat. No. 4,316,893. The formulations can also include oleic acid.

Formulation of the disclosed compositions may be achieved by conventional methods, as by the simple mixing of all components thoroughly. The artisan will appreciate that compositions containing diols other than propylene glycol and alcohols other than ethanol (i.e., 2-propanol) may find utility in transdermal anagrelide compositions as a component of the formulation. To the extent that such formulation exhibits the characteristics of the present compositions, such formulations are considered to fall within the scope of the present disclosure.

The present disclosure provides a transdermal patch formulation comprising anagrelide as an effective amount of compound of formula, from 0.1 to 10 parts by weight azone, from 30 to 69.8 parts ethanol, 29 to 50 parts by weight water, from 0 to 30 parts by weight propylene glycol, and 1 to 5 parts by weight Klucel HF. Klucel HF is a trade mark and is hereinafter referred to as Klucel HF. Preferred ranges for the formulation include from 2 to 4 parts by weight azone, from 30 to 55 parts by weight ethanol, from 0 to 20 parts by weight propylene glycol, from 35 to 45 parts water, and from 2.5 to 3.5 parts Klucel HF. One further embodiment is to omit propylene glycol from the formulation.

There is provided a transdermal formulation patch wherein an effective amount of anagrelide is intimately distributed in a matrix. One such preferred matrix is a pressure sensitive adhesive.

Further, there is provided a transdermal patch formulation comprising an effective amount of anagrelide and from about 70 to 99.8% acrylate adhesive. A preferred range of acrylic adhesive comprises from about 66 to about 99.8% by weight acrylic adhesive. A further preferred range of acrylic adhesive comprises from about 70 to about 98% by weight acrylic adhesive. Another preferred range for the acrylate adhesive is from about 80 to 98 parts by weight. The acrylate adhesive is commercially available and may be purchased for example, from the National Starch and Chemical Corporation, Bridgewater, N.J. 08807, catalog number 80-1054. The acrylate adhesive typically contains 48% solids in 33% ethyl acetate/28% heptane/34% isopropanol/5% toluene by weight. A preferred range for the acrylate adhesive is from about 80 to 98 parts by weight

Additionally, there is provided a transdermal patch formulation comprising an effective amount anagrelide, from 85 to 97 parts by weight ethanol and from 2 to 14.9 parts Klucel HF. Klucel HF is a commercially available gelling agent. For example, Klucel HF may be purchased from Aqualon. Other appropriate gelling agents can be selected by the skilled artisan. Preferred ranges for the formulation are 92 to 96 parts by weight ethanol and 2.5 to 3.5 parts Klucel HF or other appropriate gelling agent. Another preferred range for such formulations comprises from about 93 to about 95 parts by weight ethanol and from about 3 to about 3.5 parts gelling agent

Preferred transdermal patch formulations include but are not limited to a patch formulation comprising an effective amount of anagrelide, azone, ethanol, water, optionally propylene glycol and Klucel HF; anagrelide intimately distributed in a matrix; anagrelide and an acrylic adhesive; an anagrelide, ethanol, and Klucel HF; described herein.

In one embodiment, the size of the transdermal patch or application to the skin via a delivery system is from about 10cm² to about 100cm². In a further embodiment, the size of the transdermal patch or application to the skin via a delivery system is from about 30cm² to about 75cm². In a further embodiment, the size of the transdermal patch or application to the skin via a delivery system is from about 40cm² to about 60cm². In a further embodiment, the size of the transdermal patch or application to the skin via a delivery system is from about 45cm² to about 55cm². In a further embodiment, the size of the transdermal patch or application to the skin via a delivery system is from about 15cm² to about 55cm². In a further embodiment, the size of the transdermal patch or application to the skin via a delivery system is from about 20cm² to about 40cm².

Plasma levels can be determined using gas chromatography or Liquid chromatography (LCMS-MS) methods familiar to the skilled artisan. The artisan can establish the appropriate conditions for the gas chromatographic analysis.

It shall be understood that other suitable enhancers and substances beneficial to the drug substance skin flow may preferably be included in the formulations of this disclosure. Such penetration enhancers such as linalool, carvacrol, thymol, citral, menthol and t-anethole. Further examples of permeation enhancers include, but are not limited to, fatty acid esters of glycerin, such as capric, caprylic, dodecyl, oleic acids; fatty acid esters of isosorbide, sucrose, polyethylene glycol ; caproyl lactylic acid; laureth-2 ; laureth-2 acetate; laureth-2 benzoate; laureth-3 carboxylic acid ; laureth-4 ; laureth-5 carboxylic acid; oleth-2 ; glyceryl pyroglutamate oleate ; glyceryl oleate ; N-lauryl sarcosine; N-myristoyl sarcosine; N-octyl-2pyrrolidone ; lauraminopropionic acid; polypropylene glycol-4-laureth-2 ; polypropylene glycol-4-laureth-5dimethyl lauramide ; lauramide diethanolamine (DEA). Preferred enhancers include, but are not limited to, lauryl pyroglutamate (LP), glyceryl monolaurate (GML), glyceryl monocaprylate, glyceryl monocaprate, glyceryl monooleate (GMO) and sorbitan monolaurate

Disclosed is a transdermal drug delivery system comprising anagrelide, anagrelide in base form, or a pharmaceutically acceptable salt of anagrelide, and at least one dermal penetration enhancer, wherein the dermal penetration enhancer is a safe skin-tolerant ester sunscreen, and optionally at least one volatile liquid.

According to another aspect of this disclosure, there is provided a use of an effective amount of anagrelide, anagrelide in base form, or a pharmaceutically acceptable salt of anagrelide to a patient in need thereof comprising applying to a dermal surface of the patient a transdermal drug delivery system comprising anagrelide, anagrelide in base form, or a pharmaceutically acceptable salt of anagrelide, and at least one dermal penetration enhancer, wherein the dermal penetration enhancer is a safe skin-tolerant ester sunscreen, and optionally at least one volatile liquid.

In accordance with another aspect of this disclosure, there is provided a non-occlusive, percutaneous or transdermal drug delivery system comprising
(i) an effective amounts of anagrelide, anagrelide in base form, or a pharmaceutically acceptable salt of anagrelide;
(ii) at least one non-volatile dermal penetration enhancer; and
(iii) at least one volatile liquid;
wherein the dermal penetration enhancer is adapted to transport anagrelide, anagrelide in base form, or a pharmaceutically acceptable salt of anagrelide across a dermal surface when the volatile liquid evaporates, to form a reservoir or depot of a mixture comprising the penetration enhancer and the anagrelide within the surface. The dermal penetration enhancer is of low toxicity so that it is tolerated by the dermal surface.

This disclosure relates to a method of administering an effective amount of anagrelide, anagrelide in base form, or a pharmaceutically acceptable salt of anagrelide to a patient in need thereof comprising applying to a dermal surface of the patient a transdermal drug delivery system comprising.
(i) an effective amount of anagrelide, anagrelide in base form, or a pharmaceutically acceptable salt of anagrelide;
(ii) at least one non-volatile dermal penetration enhancer; and
(iii) at least one volatile liquid;
wherein the dermal penetration enhancer is adapted to transport anagrelide, anagrelide in base form, or a pharmaceutically acceptable salt of anagrelide across a dermal surface when the volatile liquid evaporates, to form a reservoir or depot ' of a mixture comprising the penetration enhancer and the anagrelide within the surface.

In one aspect, there is provided, a use in accordance with this invention, wherein administration is via the transdermal drug delivery system over an area of 5cm² to 100cm².

As described in US 6,299,900, the non-occlusive drug delivery system is preferably not supersaturated with respect to the active ingredient, in this case anagrelide, anagrelide in base form, or a pharmaceutically acceptable salt of anagrelide. As the volatile liquid evaporates, the resulting non-volatile composition is rapidly driven into the dermal surface. While it is possible that as the volatile liquid evaporates, the non-volatile dermal penetration enhancer becomes supersaturated with respect to the anagrelide, it is, however, preferred that any supersaturation does not occur before transport of the resulting non-volatile compositions across the epidermal surface has occurred.

Preferably, following application of the non-occlusive transdermal drug delivery system, the volatile component evaporates and the relevant area of skin becomes touch-dry, preferably within 10 minutes, more preferably within 3 minutes, most preferably within 1 minute.

Again, as described in US 6,299,900, preferred dermal penetration enhancers include esters of formula (I): 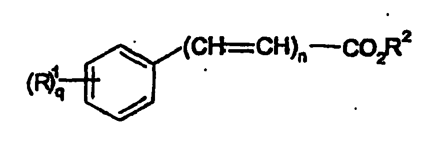 wherein
R¹ is hydrogen, lower alkyl, lower alkoxy, halide, hydroxy or NR³R⁴;
R² is a long chain alkyl;
R³ and R⁴ are each independently hydrogen, or lower alkyl, or
R³ and R⁴ together with the nitrogen atom to which they are attached form a 5- or 6-membered heterocyclic ring;
n is 0 or 1; and
q is 1 or 2.

Preferred esters of formula (I) include long chain alkyl para-aminobenzoate, long chain alkyl dimethyl-para-aminobenzoate, long chain alkyl cinnamate, long chain alkyl methoxycinnamate or long chain alkyl salicylate, for example, octyl dimethyl-para-aminobenzoate, octyl para-methoxycinnamate, octyl salicylate or isoamyl salicylate.

In addition to the dermal penetration enhancers of formula (I), known dermal penetration enhancers may be employed in the non-occlusive transdermal drug delivery system of the present dislosure.

Preferred volatile liquids of the present disclosure include safe skin-tolerant solvents such as ethanol and isopropanol. An aerosol propellant, such as dimethyl ether, may constitute a volatile liquid for the purpose of the present disclosure.

In a further embodiment, there is provided a combination useful for the treatment or prevention of thrombocythemia in which the transdermal formulation of the present invention further comprise anagrelide and at least one further therapeutic agent chosen from, hydroxyurea, P³², busulphan, aspirin, clopidogrel, dipyridamole, ticlopidine and α-interferon.

The combinations referred to above may conveniently be presented for use in the form of a pharmaceutical formulation and thus pharmaceutical formulations comprising a combination as defined above together with a pharmaceutically acceptable carrier therefore comprise a further aspect of the disclosure.

The individual components of such combinations may be administered either sequentially or simultaneously in separate or combined pharmaceutical formulations.

When the compound is used in combination with a second therapeutic agent, the dose of each compound may be either the same as or differ from that when the compound is used alone. Appropriate doses will be readily appreciated by those skilled in the art.

The ratio between the compounds of the present invention and the second therapeutic agent will be readily appreciated by those skilled in the art. For example, one may use from about 1:1 to about 1: 50 of compounds of the disclosure:second therapeutic agent. In a further embodiment one may use from about 1:1 to about 1:30 of compounds of the disclosure:second therapeutic agent. In a further embodiment, one may use from about 1:1 to about 1: 20 of compounds of the disclosure:second therapeutic agent. In a further embodiment, one may use from about 1:1 to about 1:15 of compounds of the disclosure:second therapeutic agent In a further embodiment, one may use from about 1:1 to about 1:10 of compounds of the disclosure:second therapeutic agent. In a further embodiment, one may use from about 1:1 to about 1:5 of compounds of the disclosure:second therapeutic agent. In a further embodiment, one may use from about 1:1 to about 1:3 of compounds of the disclosure:second therapeutic agent If a further therapeutic agent is added, ratios will be adjusted accordingly.

The following examples are provided to illustrate various embodiments of the present invention and shall not be considered as limiting in scope.

### EXAMPLE 1

### Transdermal formulation Free Base

A 0.5 g sample of anagrelide is dissolved in a suitable amount of ethanol (200 proof). A 0.75 g sample of azone and a 5.0 g aliquot of propylene glycol are added to the ethanol mixture with stirring. A 10 g sample of water is added to the mixture. Finally, 0.75 g of Klucel is added to the mixture and stirred until the Klucel is dispersed. The mixture is allowed to stand for 24 hours. A 2.0 g sample of the formulation prepared as described herein is dispensed by syringe into a reservoir-type transdermal adhesive system.

### EXAMPLE 2

### Transdermal Formulation without Polyethylene Glycol

A 0.5 g sample of anagrelide is dissolved suitable amount of ethanol (200 proof). A 0.79 g sample of azone is added to the ethanol mixture with stirring. An 11.29 g sample of water is added to the mixture. Finally, 0.79 g of Klucel is added to the mixture and stirred until the Klucel is dispersed. The mixture is allowed to stand for 24 hours. A 2.0 g sample of the formulation prepared as described herein is dispensed by syringe into a reservoir-type transdermal adhesive system.

### EXAMPLE 3

### Transdermal anagrelide in acrylic adhesive

A 600 mg sample of anagrelide is dissolved in 41.6 g of pressure sensitive acrylic adhesive (cat. number 80-1054, National Starch and Chemical Corporation, Bridgewater, N.J. 08807). The mixture is agitated for 2 hours on a three roller mill. The mixture is coated along the length of a 3 mil thick release liner using a knife coater providing a 20 mil gap. The 20 mil gap provides an effective 20 mil thick coating of the formulation on the release liner. The sample is allowed to air dry for 24 hours. The sample is laminated on polyester backing.

### EXAMPLE 4

### Transdermal anagrelide in Gel

A 1.0 g sample of anagrelide is dissolved in suitable amount of ethanol (200-proof). Then a 1.5 g sample of Klucel gelling agent is added to the solution and stirred until dispersed. The gel is allowed to stand for 24 hours. A 2.0 g sample of the formulation prepared as such is dispensed by syringe into a reservoir-type transdermal adhesive system.

### EXAMPLE 5

Duro-Tak 87-2287 is a solution polyacrylate adhesive available from National Starch and Chemical Co. Its monomer composition is: vinyl acetate, 2-ethylhexyl acrylate, hydroxyethyl acrylate, and glycidyl methacrylate. It contains no crosslinking agent. It is available as a 50% solids solution in ethyl acetate.

Mixtures of Duro-Tak 87-2287, 0.26% aluminum acetylacetonate crosslinker, 6% anagrelide and various permeation enhancers are prepared, each system respectively comprising one of: lauryl pyroglutamate (9 wt%), glycerol monocaprylate (10 wt%), or glycerol monocaprate (5 wt%), These mixtures are cured and cast as a 100 micron thick (wet) layer onto a 3M 1022 polyester backing and dried.

### EXAMPLE 6

Silicone 4202 is a polydimethylsiloxane adhesive from Dow Coming. It is mixed with anagrelide, 7% PVP (K30 from BASF; dissolved in n-propanol) and various enhancers, each system respectively comprising one of: lauryl pyroglutamate (9 wt%), glycerol monocaprylate (10 wt%), and glycerol monocaprate (5 wt%), These mixtures are cast as a 100 micron thick (wet) layer onto a 3M 1022 polyester backing and dried.

### EXAMPLE 7

PIB solutions are prepared by dissolving VISTANEX L100, Vistanex LM-MS-LC, and polybutene (Indopol H1900) in hexane. Suspensions of PVP-CLM, anagrelide and various enhancers in ethanol/ethyl acetate are prepared. The enhancers comprise one or more of the following; thioglycerol (2 - 4%wt.), oleic acid (4%wt.), methyl laurate (10 -15% wt.) and propylene glycol monolaurate (10% wt.) The PIB solution was added to the drug suspensions and the resulting mixtures were thoroughly blended. The mixtures are cast as a 10 mil thick (wet) layer onto release liners and dried at 70 DEG C. for min. Saranex 2015 backing is laminated to the subassembly.

### EXAMPLE 8 Comparison between the oral, intr-venous and trandermal mode of andimistration of anagrelide in the mini-pig.

The plasma level of anagrelide and metabolite A were measured in a mini-pig study following oral, intra-venous and transdermal administration of anagrelide.

The chemical structures of anagrelide and Metabolite A are shown below:

### Anagrelide:

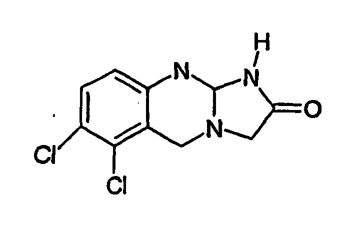

### Metabolite A

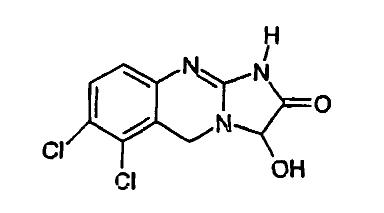

The comparative bioavailability of anagrelide following oral administration of 1mg or the dermal application of a saturated solution to ∼45cm2 surface area of the back was assessed in three minipigs. Standard AgrylinR capsules (2x0.5mg) were administered orally to fasted animals, whereas the dermal formulation was a saturated solution of the drug in 5% (v/v) oleic acid in propylene glycol.

Plasma concentrations of anagrelide and its metabolites were determined by a validated LC-MS/MS assay. Pharmacokinetic parameters were calculated by non-compartmental methods using WinNonlin.

Drug administered by either route resulted in comparable exposure to anagrelide although the concentration-time profiles were markedly different. The rate of absorption was slower and the maximum plasma concentration lower (50%) following dermal application, and in further contrast to the oral route there was only a limited decline in concentrations (50%) post-peak prior to wash-off of residual dose at 24h. Rapid decline in concentrations after wash-off confirmed the conclusion that topical absorption continued throughout the period of application.

The average dermal flux was estimated to be 197ng/cm2/h.

This table summarises the study and the results

### Studies in the mini-pig (n=3)

| Route/dose/formulation | Cmax (ng/ml) Anagrelide | Cmax (ng/ml) Metabolite A | AUC (ng.h/ml) Anagrelide | AUC (ng.h/ml) Metabolite A |
|---|---|---|---|---|
| Oral 1 mg/capsule | 1.45 | 0.75 | 10.3 | 6.8 |
| Intra-venous 1mg/propylene glycol | 87.1 | 2.79 | 50.7 | 7.1 |
| Dermal 5% OA in PG | 0.69 | 0.27 | 10.8 | 4.1 |

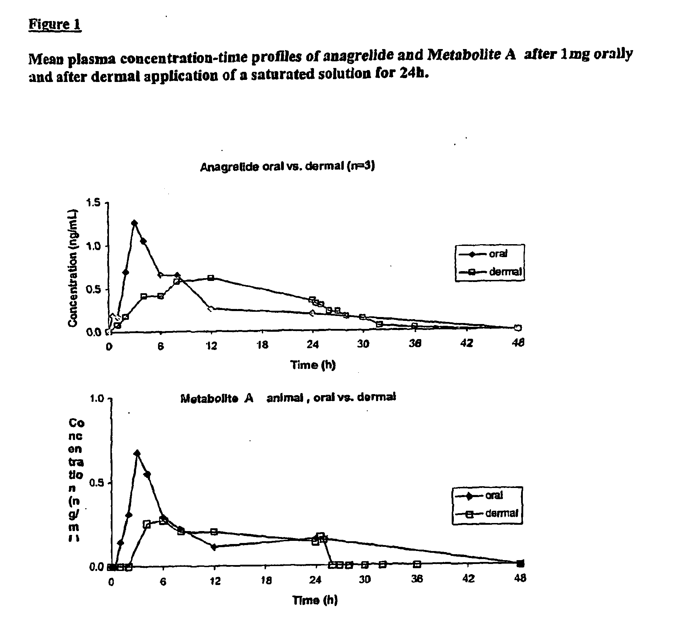

The results are also depicted in Figure 1.

### EXAMPLE 9 PDEIII activity of metabolite A

Inhibition of PDE III, which is present in myocardium, causes an increase in both the force and rate of cardiac contractility. These are undesirable side effects for a platelet reducing agent.

The PDE III activity of both anagrelide and metabolite A was evaluated by standard methods. Metabolite A. is 40X more potent then anagrelide.

### EXAMPLE 10 Pharmacokinetics studies on anagrelide

Previous limited clinical PK studies have shown that after oral administration of anagrelide there is a potential for significant exposure to the potent cardioactive metabolite A. While this compound undoubtedly contributes to the therapeutic platelet lowering action of anagrelide, with which it is equipotent, it is some 40 times more potent as a cardiovascular agent. A studies in total of 38 healthy male volunteers provides evidence of the extent of exposure to this metabolite as shown in the table below:

### Summary of mean pharmacokinetic parameters of anagrelide in volunteers after a single dose of the drug

| **Compound** | **Mean Pharmacokinetic Parameters ± RSD (%)** | | | | |
|---|---|---|---|---|---|
| | **Dose/N** | **AUC₀₋ᵢₙ ± PSD (%)** | **Cₘₐₓ ± RSD (%)** | **Tₘₐₓ ± RSD (%)** | **t_{1/2} ± RSD (%)** |
| | | **(ng·h/mL)** | **(ng/mL)** | **(hours)** | **(hours)⁾** |
| Anagrelide | 1 mg (38) | 11.1 ± 37.6 | 4.99 ± 74.4 | 1.3 ± 53.8 | 1.5 ± 49.8 |
| Metabolite A | 1 mg (38) | 18.0 ± 35.6 | 5.47 ± 56.9 | 1.28 ± 58.1 | 2.5 ± 28.7 |

Furthermore, data in patients suffering from myeloproliferative disease has shown at steady state even higher relative exposure to this metabolite compared to the parent drug occurs the ratio of metabolite to drug AUC being close to 3:1 This is shown in the table below:

### Summary of mean pharmacokinetic parameters of BCH24426 in patients following multiple of the drug

| **Compound** | **Mean Pharmacokinetic Parameters ± RE** | | | | |
|---|---|---|---|---|---|
| | N | ***AUC₀₋ₜ ± RE** | **Cₘₐₓ± RE** | **Tₘₐₓ ± RE** | **t_{1/2} ± RE** |
| | | **(ng·h/mL)** | **(ng/mL)** | **(hours)** | **(hours)⁾** |
| Anagrelide | 18 | 18.64(5.28) | 5.31(1.33) | 2.00(0.32) | 2.89(0.73) |
| Metabolite A | 18 | 48.89(17.90) | 7.61(1.63) | 2.25(0.28) | 4.27(0.56) |

| | | | | | |
|---|---|---|---|---|---|
| *AUC over dosage interval | | | | | |

### EXAMPLE 11 cardiovascular studies on Metabolite A

Earlier *in vitro* studies have already demonstrated the comparatively greater potency of metabolite A (40 fold) relative to anagrelide as an inhibitor of PDEIII. A study was conducted in a large group of dogs comparing metabolite A with the standard reference inotrope milrinone. A total of 12 animals have been used in this study which has shown metabolite A to be qualitatively like milrinone in it effects on the cardiovascular system but very considerably more potent. The essential conclusions from this work areas follows:
• Metabolite A and milrinone cause a dose-dependent increase in **heart rate;** the mean maximum *increase* in the Metabolite A group was ∼66 b.p.m. and that for milrinone is ∼76.
• Metabolite A and milrinone produce a dose-dependent decrease **in mean blood** pressure, with a maximum decrease of about 30 mmHg, although Metabolite A was 10 x more potent than milrinone.
o Metabolite A increased **(+)dP/dt max**( a measure of contractility) which was well sustained and largely dose-dependent;. Milrinone causes immediate, dose-dependent increases in (+)dP/dt max, but they were not well sustained. The picture with **(+)dP/dt40** was broadly similar.
• Neither compound had a profound effects on **femoral, carotid or renal blood flows** i.e. blood flow to these vascular beds was largely sustained *despite* the fall in blood pressure, implying an increase in vascular conductance in these beds.

These cardiovascular effects are those to be expected of a PDEIII inhibitor and are consistent with the adverse event profile seen in some patients treated with anagrelide and support the belief that this metabolite is indeed responsible for those observed side effects. Thus reduction of the proportion of this metabolite by transdermal administration is expected to significantly reduce the side effect profile of the drug.

### EXAMPLE 11 Further non-clinical studies to assess the effectiveness of continuous low-level exposure to anagrelide

Earlier studies in the minipig have shown that transdermal application of anagrelide leads to lower but sustained exposure to the drug and much reduced proportion of the metabolite compared to oral administration which is potentially of benefit in minimising the CVS effects of the drug itself. However conformation was required that the therapeutic response i.e. platelet lowering, would not be adversely affected.

In order to confirm that lower continuous level exposure - in contrast to the regular plasma higher peaks and troughs associated oral administration - were still effective in reducing blood platelets. It has been calculated that the maximum likely flux rate through human skin could give rise to a Cav of ∼3-4ng/ml. It was therefore important to demonstrate that at this level adequate reduction in megakaryocyte formation could be achieved.

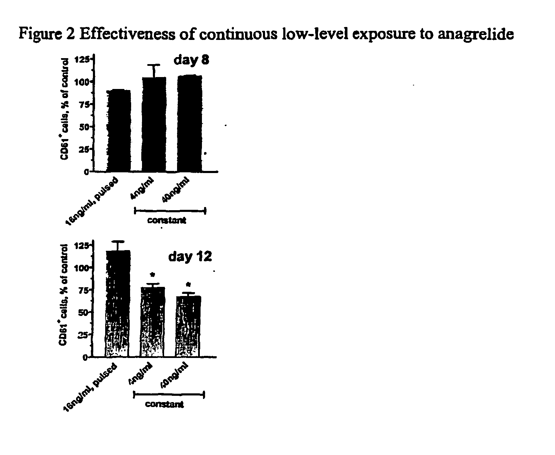

In order to mimic a transdermal delivery of anagrelide in culture, CD34⁺ cells that had been expanded for 4 days in the presence of 40 ng/ml thrombopoeitin were treated for 8 additional days by continuous exposure to a concentration of 4 ng/ml anagrelide (∼13 nM). Cells were harvested for analysis after 4 or 8 days of the initiation of drug treatment (day-8 and day-12 cultures, respectively). As shown in Figure 2 in day-8 cultures no effect of anagrelide could be detected. In contrast in day-12 cultures anagrelide treatment caused a statistically significant reduction in the number of megakaryocytes (77 ± 5 % of control, p= 0.038, n =3). Similarly, in parallel cultures, a single dose of 40 ng/ml anagrelide (∼133 nM) showed no effect on day-8 cultures but caused a significant inhibition in day 12-cultures (68 ± 4 % if control, *p*= 0.015, n=3)..

These results confirm the likely effectiveness of continuous low-level exposure in man (to just 4ng/ml) in reducing megakaryocyte production and thereby platelet numbers.

### Example 12 Permeation studies of anagrelide from saturated solutions of the drug in different formulations through human epidermis

### METHODS

### FORMULATIONS:

Saturated solutions of anagrelide were prepared in:
1.5% lauryl alcohol in isopropyl myristate (LA in IPM)
2. 2% oleic acid in propylene glycol (OA in PG)
3. 0.5% oleic acid in propylene glycol
4. 5% glyceryl monooleate in isopropyl myristate (GMO in IPM)
5. 5% glyceryl laurate in isopropyl myristate (GLA in IPM)
6. Formulation 1:
   ➢ Labrasol 53.5 %
   ➢ Plurol Oleique 13.4 %
   ➢ Labrafac Lipophile 15 %
   ➢ Propylene glycol 18%
7. Formulation 2:
   ➢ Labrafil M 1944CS 13.2 %
   ➢ Labrafac Lipophile 31.8 %
   ➢ Labrasol 32.5 %
   ➢ Flurol oleique 13.5 %
   ➢ Water 9.0
8. Transcutol
9. Isopropyl myristate (IPM)
10. Triacetin
11.5% oleic acid (OA) in propylene glycol (PG)
12. 70:30 (v/v) dimethyl sulfoxide: propylene glycol

### Analytical Method Validation

An analytical method using HPLC with UV detection was established for anagrelide. Six point calibration curves were generated over the range 0.2-2 µg/ml were generated for each analytical run and the precision confirmed on each occasion by the making at least 7 replicate injections of the highest standard. Details of the equipment and methods used are given below.

### HPLC Equipment

**Column:** Apex reverse phase ODS 5µm packed column (250 x 4.6 mm)
**Pump:** Thermo Separation Products Spectra Series P100
**Autosampler:** Thermo Separation Products Spectra SERIES AS100
**Detector:** Thermo Separation Products SpectraSERIES UV100
**Integrator:** Thermo Separation Products ChromJet

### Chromatographic conditions

*Mobile Phase:* Acetonitrile - 0.025M phosphate (KH₂PO₄) (50:50)

The mobile phase was degassed through a Millipore filter prior to use.

Column temperature: Ambient

| | |
|---|---|
| Flow Rate: | 0.5mL per minute |
| Injection volume: | 20 µL |
| Wavelength: | 255nm |
| Retention time: | ∼6.8 minutes |

pH Meter: Electronics Instruments Limited (Kent) Model no. 7065.

### Preparation of standard solutions for calibration

A stock solution containing 10mg of anagrelide in 500ml acetonitrile: water (60:40) was used for to generate the calibration standards. The temperature was raised to about 50°C to ensure complete dissolution. Dilutions were made from the stock solution to give concentrations in the range 0.2-2 µg/mL. The standards were analyzed using the HPLC procedure mentioned above. A correlation coefficient of 0.9991 was obtained.

The reliability of the HPLC system was established before every run by analyzing the same concentration of the drug seven times. Typically a coefficient of variation of around 0.7% was obtained.

### Preparation of the human epidermis

Human epidermis was prepared by the heat separation technique *(*A.M. Kligman and E Christophers, Preparation of isolated sheets of human stratum corneum, Arch Dermatol., vol. 88, 70-73 (1963*).* Water was heated to 60°C on a hotplate and the skin was immersed in the water at this temperature for 1 minute. The skin was then removed from the water and the epidermis carefully peeled off using blunt tweezers. Care was taken not to introduce any holes in this process. The epidermal tissue was placed on a filter paper stratum corneum uppermost. The samples were then stored in freezer.

### Diffusion cells

Franz-type horizontal glass diffusion cells were used. The receptor medium was thermostatted at 37°C, to represent body temperature. There will be a temperature gradient across the membrane and applied solution, but this should simulate 'in use' conditions. Under these conditions the surface temperature of the skin was 32°C.

The skin samples were thawed overnight before use. The epidermal membranes were cut to size and were placed between the two halves of the cells. High vacuum grease was used to seal the two compartments. The cell was clamped using a metal holder. The receptor arm was closed using glass caps to prevent evaporation. The donor compartment was occluded to prevent any donor solution evaporation

The receptor medium was first introduced and equilibrated for 1h. 1mL of the saturated solution with excess drug was applied in the donor compartment. Excess drug was used to ensure there is no depletion of the drug during the course of the experiment. The starting time was taken as the time at which the solutions were applied.

At set sampling points (12, 24, 30, 36 and 48 hours), 200µL of the receptor phase from each diffusion cell was removed for analysis of the anagrelide and replaced by an equivalent of fresh receptor phase solution pre-thermostatted to 37°C.

For each solution, six replicates were tested. A control (without any formulation applied in the donor compartment) was also examined.

### RESULTS

Anagrelide was found to permeate, to a limited extent, from all the solutions. The permeation at 24h was found to be highest for anagrelide in ethanol (0.9µg/cm²) followed by the drug in propylene glycol (0.5µg/cm²) and then in glycerol (0.04µg/cm²)_{.}

Anagrelide was found to permeate, to a limited extent, from all the solutions. The permeation at 24h was found to be highest for anagrelide in 2% OA in PG (8.9µg/cm²), 5% GMO in IPM (5.7µg/cm²) and 5% GLA in IPM (5.2µg/cm²). The permeation from the suggested 'gold standard', anagrelide in 70:30 DMSO: PG, was similar to the highest permeation rates achieved (8.4µg/cm²). Results are shown in the following table:

| **Anagrelide permeated at 24hours from different solutions** | | |
|---|---|---|
| | | |
| **Solvent** | **Amt permeated (µg/cm²)** | **SD** |
| **Glycerol** | 0.04 | 0.03 |
| **Ethanol** | 0.9 | 0.5 |
| **PG** | 0.5 | 0.4 |
| **IPM** | 1.5 | 0.7 |
| **Transcutol** | 0.0 | 0 |
| **Triacetin** | 0.0 | 0 |
| **5% OA in PG** | 4.4 | 1 |
| **2%OA in PG** | 8.9 | 1.5 |
| **0.5% OA in PG** | 0.5 | 0.3 |
| **5% GMO in IPM** | 5.7 | |
| **5% GLA in IPM** | 5.2 | 1.1 0.5 |
| **5% LA in IPM** | 0.5 | 0.2 |
| **Formulation 1** | 2.1 | 0.4 |
| **Formulation 2** | 1.5 | 0.6 |
| **70:30 DMSO:PG** | 8.4 | 3 |

Anagrelide was found to permeate, to a limited extent, from all the solutions. The amount permeated at 24h was found to be highest for anagrelide in 5% OA in PG (4.4 µg/cm²) followed by the drug in IPM (1.5µg/cm²). The results are shown in the following table:

| **Solvent** | **Amount permeated at 24.h (µg/cm²)** | **SD** |
|---|---|---|
| **Glycerol** | 0.04 | 0.03 |
| **Ethanol** | 0.9 | 0.5 |
| **PG** | 0.54 | 0.4 |
| **IPM** | 1.5 | 0.7 |
| **Transcutol** | 0 | 0 |
| **Triacetin** | 0 | 0 |
| **5% OA in PG** | 4.4 | 1 |

## Claims

1. Use of an anagrelide agent in the form selected from anagrelide, anagrelide in base form, or a pharmaceutically acceptable salt of anagrelide, in the preparation of a medicament for use in the treatment of thrombocythemia in a subject, wherein the mode of administration is by transdermal administration.

2. Use according to claim 1, wherein the anagrelide agent is administered by contacting an area of skin with the anagrelide agent formulated with a skin permeation enhancer.

3. Use according to claim 1, wherein the anagrelide agent is in the form of a reservoir formulation; or in the form of a single layer formulation comprising the anagrelide agent and at least one adhesive; or in the form of a multiple layer formulation wherein at least one layer of the multiple layer formulation comprises the anagrelide agent and at least one adhesive; or in the form of a matrix formulation.

4. Use according to claim 1, wherein the thrombocythemia is associated with essential thrombocythemia (ET), chronic myologenous leukemia (CML), polycythemia vera (PV), agnogenic myeloid metaplasia (AMM) or sickle cell anemia (SCA).

5. Use according to claim 1, wherein the anagrelide agent is administered in an amount of 0.01 to 20 mg/kg/day.

6. Use according to claim 1, wherein the anagrelide agent is in the form of a composition which further comprises at least one skin permeation enhancer.

7. Use according to claim 6, wherein the at least one penetration enhancer is linalool, carvacrol, thymol, citral, menthol or t-anethole.

8. Use according to claim 1, wherein administration is via a transdermal patch having a single-layer drug-in-adhesive system comprising a composition containing the anagrelide agent, any optional excipients, and at least one skincontacting adhesive, which is combined with a single backing film;
or administration is via a transdermal patch having a multi-layer drug-in-adhesive system wherein:
(a) the system comprises at least two distinct layers comprising the anagrelide agent and at least one adhesive, and a membrane between the at least two layers or
(b) the system comprises at least two distinct layers comprising the anagrelide agent and at least one adhesive, and a single backing film; or
administration is via a transdermal patch having a reservoir transdermal system comprising a liquid compartment containing a solution or suspension of the anagrelide agent, a release liner, and between the release liner and the liquid compartment, a semi-permeable membrane and at least one adhesive;
or administration is via a transdermal patch having a matrix system comprising a semisolid matrix containing a solution or suspension of the anagrelide agent which is in direct contact with a release liner, and a skin adhesion component incorporated in an overlay which forms a concentric configuration around the semisolid matrix; or
administration is via a transdermal patch containing the anagrelide agent intimately distributed in a matrix.

9. Use according to claim 1, wherein administration is via a transdermal patch containing 1 mg to 100 mg of the anagrelide agent per patch.

10. Use according to claim 1, wherein the transdermal patch contains an acrylic adhesive.

11. Use according to claim 10, wherein the composition contains 66 to 99.8% by weight acrylate adhesive.

12. Use according to claim 1, wherein the administration is via a transdermal patch containing an amount of the anagrelide agent, azone, ethanol, water, optionally propylene glycol and hydroxypropylcellulose gelling agent Klucel HF™.

13. Use according to claim 12, wherein administration is via a transdermal patch containing an amount of the anagrelide agent, 0.1 to 10 parts by weight azone, from 30 to 69.8 parts ethanol, 29 to 50 parts by weight water, from 0 to 30 parts by weight propylene glycol, and 1 to 5 parts by weight hydroxypropylcellulose gelling agent Klucel HF™.

14. Use according to claim 1, wherein administration is via a transdermal patch containing the anagrelide agent, ethanol, and hydroxypropylcellulose gelling agent Klucel HF™.

15. Use according to claim 14, wherein administration is via a transdermal patch containing an amount of the anagrelide agent, 85 to 97 parts by weight ethanol and 2 to 14.9 parts hydroxypropylcellulose gelling agent Klucel HF™

16. Use according to claim 1, wherein administration is via a transdermal patch having an area of 5 cm² to 100 cm².

## Patentansprüche

1. Einsatz eines Anagrelid-Wirkstoffs in einer Form, die ausgewählt wird aus Anagrelid, Anagrelid in Basisform oder einem pharmazeutisch verträglichen Salz von Anagrelid, für die Herstellung eines Arzneimittels zum Einsatz in der Behandlung von Thrombozythämie in einem Patienten, wobei es sich bei dem Modus der Verabreichung um eine transdermale Verabreichung handelt.

2. Einsatz nach Anspruch 1, wobei der Anagrelid-Wirkstoff verabreicht wird durch Kontaktieren eines Hautbereichs mit dem Anagrelid-Wirkstoff, der mit einer die Hautdurchdringung fördernden Substanz formuliert ist.

3. Einsatz nach Anspruch 1, wobei der Anagrelid-Wirkstoff in Form einer Speicherzubereitung vorgesehen ist; oder in Form einer einlagigen Zubereitung, die den Anagrelid-Wirkstoff und mindestens eine Adhäsionssubstanz umfasst; oder in Form einer mehrlagigen Zubereitung, wobei zumindest eine Lage der mehrlagigen Zubereitung den Anagrelid-Wirkstoff und mindestens eine Adhäsionssubstanz umfasst; oder in Form einer Matrixzubereitung.

4. Einsatz nach Anspruch 1, wobei die Thrombozythämie essentieller Thrombozythämie (ET), chronisch myeloischer Thrombozythämie (CML), Polyzythämie vera (PV), agnogener myeloischer Metaplasie (AMM) oder Sichelzellenanämie (SCA) zugeordnet ist.

5. Einsatz nach Anspruch 1, wobei der Anagrelid-Wirkstoff in einer Menge von 0,01 bis 20 mg/kg/Tag verabreicht wird.

6. Einsatz nach Anspruch 1, wobei der Anagrelid-Wirkstoff in Form einer Zusammensetzung vorgesehen ist, die ferner mindestens eine die Hautdurchdringung fördernde Substanz umfasst.

7. Einsatz nach Anspruch 6, wobei es sich bei der mindestens einen die Durchdringung fördernden Substanz um Linalool, Carvacrol, Thymol, Citral, Menthol oder t-Anethol handelt.

8. Einsatz nach Anspruch 1, wobei die Verabreichung über ein transdermales Pflaster erfolgt, das ein einlagiges Arzneimittel-in-Adhäsionssubstanz-System aufweist, das eine Zusammensetzung umfasst, die den Anagrelid-Wirkstoff, etwaige optionale Arzneimittelträger und mindestens eine die Haut berührende Adhäsionssubstanz enthält, in Kombination mit einer einzelnen Trägerfolie; oder wobei die Verabreichung über ein transdermales Pflaster erfolgt, das ein mehrlagiges Arzneimittel-in-Adhäsionssubstanz-System aufweist, wobei:
(a) das System mindestens zwei unterschiedliche Lagen umfasst, welche den Anagrelid-Wirkstoff und mindestens eine Adhäsionssubstanz umfassen, und eine Membran zwischen den mindestens zwei Lagen; oder
(b) das System mindestens zwei unterschiedliche Lagen umfasst, welche den Anagrelid-Wirkstoff und mindestens eine Adhäsionssubstanz umfassen, und eine einzelne Trägerfolie; oder
wobei die Verabreichung über ein transdermales Pflaster erfolgt, das ein transdermales Speichersystem aufweist, das eine Flüssigkeitskammer umfasst, die eine Lösung oder eine Suspension des Anagrelid-Wirkstoffs, eine Abziehschicht und zwischen der Abziehschicht und der Flüssigkeitskammer eine halbdurchlässige Membran umfasst sowie mindestens eine Adhäsionssubstanz;
oder wobei die Verabreichung über ein transdermales Pflaster mit einem Matrixsystem erfolgt, das eine halbfeste Matrix umfasst, die eine Lösung oder eine Suspension des Anagrelid-Wirkstoffs enthält, die sich in direktem Kontakt mit einer Abziehschicht befindet, und mit einer Hautadhäsionskomponente, die in einen Überzug integriert ist, der eine konzentrische Konfiguration um die halbfeste Matrix bildet; oder
wobei die Verabreichung über ein transdermales Pflaster erfolgt, das den Anagrelid-Wirkstoff eng verteilt in einer Matrix enthält.

9. Einsatz nach Anspruch 1, wobei die Verabreichung über ein transdermales Pflaster erfolgt, das 1 mg bis 100 mg des Anagrelid-Wirkstoffs je Pflaster enthält.

10. Einsatz nach Anspruch 1, wobei das transdermale Pflaster eine Acryl-Adhäsionssubstanz enthält.

11. Einsatz nach Anspruch 10, wobei die Zusammensetzung zwischen 66 und 99,8 Gewichtsprozent Acrylat-Adhäsionssubstanz enthält.

12. Einsatz nach Anspruch 1, wobei die Verabreichung über ein transdermales Pflaster erfolgt, das eine Menge des Anagrelid-Wirkstoffs, Azon, Ethanol, Wasser, optional Propylenglykol und dem Hydroxypropylzellulose-Gelbildner Klucel HF™ enthält.

13. Einsatz nach Anspruch 12, wobei die Verabreichung über ein transdermales Pflaster erfolgt, das eine Menge des Anagrelid-Wirkstoffs enthält, 0,1 bis 10 Gewichtsanteile Azon, 30 bis 69,8 Teile Ethanol, 29 bis 50 Gewichtsanteile Wasser, 0 bis 30 Gewichtsanteile Glykol und 1 bis 5 Gewichtsanteile des Hydroxypropylzellulose-Gelbildners Klucel HF™.

14. Einsatz nach Anspruch 1, wobei die Verabreichung über ein transdermales Pflaster erfolgt, das den Anagrelid-Wirkstoff, Ethanol und den Hydroxypropylzellulose-Gelbildner Klucel HF™ enthält.

15. Einsatz nach Anspruch 14, wobei die Verabreichung über ein transdermales Pflaster erfolgt, das eine Menge des Anagrelid-Wirkstoffs, 85 bis 97 Gewichtsanteile Ethanol und 2 bis 14,9 Gewichtsanteile des Hydroxypropylzellulose-Gelbildners Klucel HF™ enthält.

16. Einsatz nach Anspruch 1, wobei die Verabreichung über ein transdermales Pflaster mit einer Fläche von 5 cm² bis 100 cm² erfolgt.

## Revendications

1. Utilisation d'un agent à base d'anagrélide sous la forme choisie parmi l'anagrélide, l'anagrélide sous une forme basique, ou un sel pharmaceutiquement acceptable de l'anagrélide, dans la préparation d'un médicament à utiliser pour le traitement d'une thrombocytémie chez un sujet, dans laquelle le mode d'administration est par administration transdermique.

2. Utilisation selon la revendication 1, dans laquelle l'agent à base d'anagrélide est administré par mise en contact d'une zone de la peau avec l'agent à base d'anagrélide formulé avec un agent favorisant la pénétration dans la peau.

3. Utilisation selon la revendication 1, dans laquelle l'agent à base d'anagrélide est sous la forme d'une formulation à réservoir ; ou sous la forme d'une formulation à couche unique comprenant l'agent à base d'anagrélide et au moins un adhésif ; ou sous la forme d'une formulation à couches multiples dans laquelle au moins une couche de la formulation à couches multiples comprend l'agent à base d'anagrélide et au moins un adhésif ; ou sous la forme d'une formulation matricielle.

4. Utilisation selon la revendication 1, dans laquelle la thrombocytémie est associée à une thrombocytémie essentielle (TE), à une leucémie myéloïde chronique (LMC), à une maladie de Vaquez, à une métaplasie myéloïde agnogénique (MMA) ou à une anémie à cellules falciformes (ACF).

5. Utilisation selon la revendication 1, dans laquelle l'agent à base d'anagrélide est administré dans une quantité de 0,01 à 20 mg/kg/jour.

6. Utilisation selon la revendication 3, dans laquelle l'agent à base d'anagrélide est sous la forme d'une composition qui comprend en outre au moins un agent favorisant la pénétration dans la peau.

7. Utilisation selon la revendication 11, dans laquelle l'agent favorisant la pénétration dans la peau est le linalol, le carvacrol, le thymol, le citral, le menthol ou le t-anéthole.

8. Utilisation selon la revendication 3, dans laquelle l'administration s'effectue par l'intermédiaire d'un patch transdermique ayant un système à couche unique de médicament dans un adhésif comprenant une composition contenant l'agent à base d'anagrélide, tout excipient facultatif, et au moins un adhésif de contact avec la peau, qui est combiné avec un film support unique ; ou
l'administration s'effectue par l'intermédiaire d'un patch transdermique ayant un système à couches multiples de médicament dans un adhésif dans lequel :
(a) le système comprend au moins deux couches distinctes comprenant l'agent à base d'anagrélide et au moins un adhésif, et une membrane entre les au moins deux couches ou
(b) le système comprend au moins deux couches distinctes comprenant l'agent à base d'anagrélide et au moins un adhésif, et un film support unique ; ou
l'administration s'effectue par l'intermédiaire d'un patch transdermique ayant un système transdermique à réservoir comprenant un compartiment de liquide contenant une solution ou une suspension de l'agent à base d'anagrélide, un revêtement anti-adhésif, et entre le revêtement anti-adhésif et le compartiment de liquide, une membrane semi-perméable et au moins un adhésif ;
ou l'administration s'effectue par l'intermédiaire d'un patch transdermique ayant un système matriciel comprenant une matrice semi-solide contenant une solution ou une suspension de l'agent à base d'anagrélide qui est en contact direct avec un revêtement de décollement et un composant d'adhésion à la peau incorporé dans une surcouche qui forme une configuration concentrique autour de la matrice semi solide ; ou
l'administration s'effectue par l'intermédiaire d'un patch transdermique contenant l'agent à base d'anagrélide distribué de façon intime dans une matrice.

9. Utilisation selon la revendication 3, dans laquelle l'administration s'effectue par l'intermédiaire d'un patch transdermique contenant 1 mg à 100 mg de l'agent à base d'anagrélide par patch.

10. Utilisation selon la revendication 3, dans laquelle le patch transdermique contient un adhésif acrylique.

11. Utilisation selon la revendication 15, dans laquelle la composition contient de 66 à 99,8 % en poids d'un adhésif acrylate.

12. Utilisation selon la revendication 1, dans laquelle l'administration s'effectue par l'intermédiaire d'un patch transdermique contenant une quantité de l'agent à base d'anagrélide, de l'azone, de l'éthanol, de l'eau, en option du propylène glycol et de l'agent gélifiant hydroxypropylcellulose Klucel HF™.

13. Utilisation selon la revendication 12, dans laquelle l'administration s'effectue par l'intermédiaire d'un patch transdermique contenant une quantité de l'agent à base d'anagrélide, de 0,1 à 10 parties en poids d'azone, de 30 à 69,8 parties d'éthanol, de 29 à 50 parties d'eau, de 0 à 30 parties en poids de propylène glycol et de 1 à 5 parties en poids d'agent gélifiant hydroxypropylcellulose Klucel HF™.

14. Utilisation selon la revendication 1, dans laquelle l'administration s'effectue par l'intermédiaire d'un patch transdermique contenant l'agent à base d'anagrélide, de l'éthanol, et de l'agent gélifiant hydroxypropylcellulose Klucel HF™.

15. Utilisation selon la revendication 14, dans laquelle l'administration s'effectue par l'intermédiaire d'un patch transdermique contenant l'agent à base d'anagrélide, de 85 à 97 parties en poids d'éthanol et de 2 à 14,9 parties d'agent gélifiant hydroxypropylcellulose Klucel HF™.

16. Utilisation selon la revendication 1, dans laquelle l'administration s'effectue par l'intermédiaire d'un patch transdermique ayant une surface de 5 cm² à 100 cm².
